# DEMANDE DE BREVET EUROPEEN

(11) **EP 1 389 614 A1**
(43) Date de publication de la demande: **18.02.2004**
(21) Numéro de dépôt: 02356159.0
(22) Date de dépôt: 12.08.2002
(51) Int. Cl.: C07D 213/61, A01N 43/40

(54) **Nouveaux dérivés de N-[2-(2-pyridyl)éthyl]benzamide comme fongicides**

(71) Demandeur: Bayer CropScience S.A., 69009 Lyon (FR)
(72) Inventeur: Mansfield, Darren James, 69004 Lyon (FR); Cooke, Tracey, St Albans AL2 3SN (GB); Thomas, Peter Stanley, Cambridge CB1 6LW (GB); Vors, Jean-Pierre, 69009 Lyon (FR); Coqueron, Pierre-Yves, 69006 Lyon (FR); Briggs, Geoffrey Gower, Harpenden, Herts AL5 5QR (GB); Lachaise, Hélène, 69009 Lyon (FR)
(74) Mandataire: Mérigeault, Shona

(57) **Abrégé**

Composé de formule générale (I) 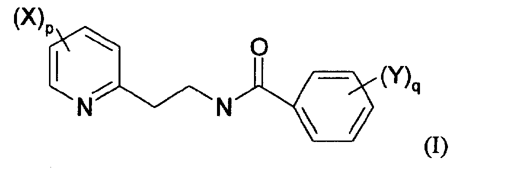 dans laquelle :
- p est un entier égal à 1, 2, 3 ou 4;
- q est une entier égal à 1, 2, 3, 4 ou 5;
- chaque substituant X est choisi indépendamment des autres dans le groupe consistant en halogène, alkyle et halogénoalkyle, l'un au moins des substituant étant un halogénoalkyle;
- chaque substituant Y est choisi indépendamment des autres dans le groupe consistant en halogène, alkyle, halogénoalkyle, alkoxy, phenoxy, alkylthio, dialkylamino, acyle, cyano, nitro, alkylsulphonyle, phénylsulphonyle, benzylsulphonyle et S-phényle substitué par un halogène;
à l'exception du N-{[3-chloro-5-(trifluorométhyl)-2-pyridinyl] éthyl}-2,6-dichlorobenzamide.

Procédé de préparation du composé de formule générale (I).

Composition fongicide comprenant le composé de formule générale (I).

Méthode traitement de maladies phytopathogènes.

## Description

La présente invention concerne de nouveaux composés fongicides, leur procédé de préparation, les compositions fongicides comprenant ces composés ainsi que leur utilisation dans le domaine de l'agriculture en tant que fongicides.

La demande de brevet WO 01/11965 décrit une famille large de composés fongicides de formule générale englobant les composés de la présente invention. Cependant, lesdits composés ne sont pas décrits dans cette demande de brevet et leur activité en tant que fongicide n'a pas été testée.

Il est néanmoins toujours utile dans le domaine de l'agriculture d'utiliser des composés plus actifs que ceux déjà connus par l'homme de l'art dans le but de diminuer les quantités de matière active à utiliser par l'agriculteur tout en maintenant une efficacité au moins équivalente aux composés déjà connus.

Il a maintenant été découvert qu'un certain nombre de composés, sélectionnés dans une famille large de composés, possédaient les avantages mentionnés ci-avant.

La présente invention a donc pour objet une famille de composés fongicides de formule générale (I) : 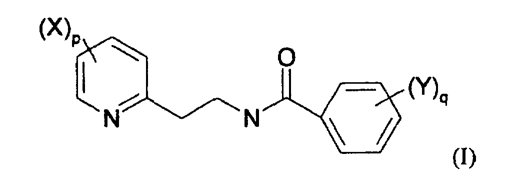 dans laquelle :
- p est un entier égal à 1, 2, 3 ou 4;
- q est une entier égal à 1, 2, 3, 4 ou 5;
- chaque substituant X est choisi indépendamment des autres dans le groupe consistant en halogène, alkyle et halogénoalkyle, l'un au moins des substituants étant un halogénoalkyle;
- chaque substituant Y est choisi indépendamment des autres dans le groupe consistant en halogène, alkyle, halogénoalkyle, alkoxy, phenoxy, alkylthio, dialkylamino, acyle, cyano, nitro, alkylsulphonyle, phénylsulphonyle, benzyisulphonyle et S-phényle substitué par un halogène;
à l'exception du N-{[3-chloro-5-(trifluorométhyl)-2-pyridinyl] éthyl}-2,6-dichlorobenzamide.

Dans le cadre de la présente invention, les substituants X de la 2-pyridine et les substituants Y du cycle benzénique seront indexés afin de faciliter la compréhension. Ainsi par exemple, si p est égal à 2 et q est égal à 1, les substituants dits "X" seront désignés par X¹ et X² et le substituant dit "Y" sera désigné par Y¹.

Au sens de la présente invention, chacun des radicaux alkyles ou acyles présents dans la molécule contient de 1 à 10 atomes de carbone, préférentiellement de 1 à 7 atomes de carbone, plus préférentiellement de 1 à 5 atomes de carbone, et peut être linéaire ou ramifié.

Les composés de formule générale (I) selon la présente invention possèdent préférentiellement les caractéristiques suivantes, prises isolément ou en combinaison :
- p est choisi égal à 2, les substituants X¹ et X² étant positionnés comme suit : 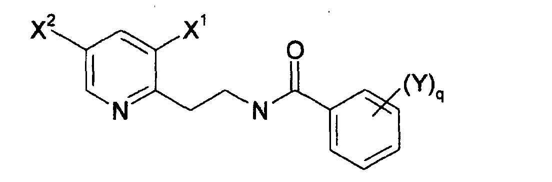
- q est choisi égal à 1 ou 2, le(s) substituant(s) Y étant positionné(s) en position ortho du cycle benzénique.

Une sous famille préférée de composés selon l'invention est constituée par les composés répondant à la formule générale (I'): 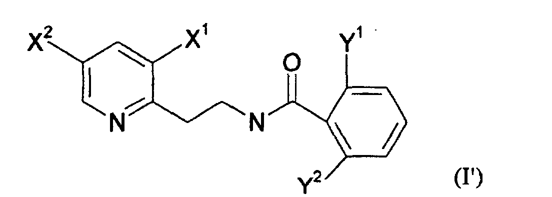 les substituants X et Y étant tels que définis précédemment. De manière encore préférée, X¹ est choisi comme étant un halogène et X² est choisi comme étant un halogénoalkyle.

Une autre sous-famille préférée de composés selon l'invention est constituée par les composés répondant à la formule générale (I"): 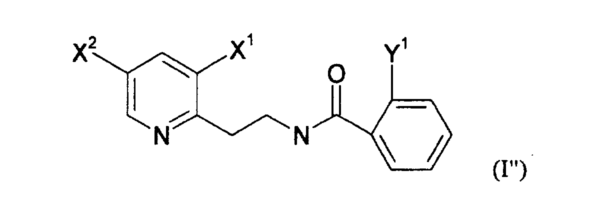 les substituants X et Y étant tels que définis précédemment. De manière encore préférée, les composés de formule générale (I") selon la présente invention possèdent les caractéristiques suivantes, prises isolément ou en combinaison :
- X¹ est choisi comme étant un halogène et X² est choisi comme étant un halogénoalkyle ;
- Y¹ est choisi comme étant un halogénoalkyle.
   De manière tout à fait préférée, les substituants X¹, X² et Y¹ sont définis comme étant respectivement Cl, CF₃ et CF₃.

La présente invention a également pour objet un procédé de préparation du composé de formule générale (I). Ainsi, la présente invention fournit un procédé de préparation des composés décrits précédemment selon les étapes suivantes :
- une première étape consistant à faire réagir en présence d'une base en solvant polaire aprotique, un composé de formule générale (Ia) pour le substituer sélectivement en position 2 :
   * soit par un groupement de type cyanoacétate d'alkyle (NC-CH₂-CO₂Alk) pour conduire à un composé de formule générale (Ib) selon le schéma réactionnel suivant : 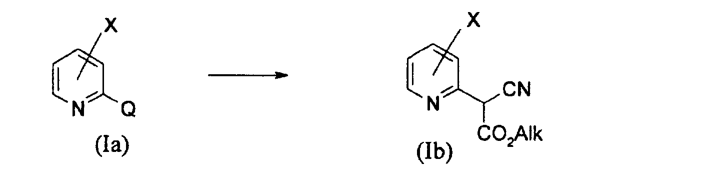
où :
- X est tel que défini précédemment;
- Alk représente un radical alkyle;
- Q est un radical nucléofuge, préférentiellement choisi comme étant un halogène ou le trifluorométhanesulfonate; le composé de formule générale (Ib) ainsi obtenu étant alors décarboalkoxylé en présence d'un halogénure alcalin tel que Li-Halogène, K-Halogène ou Na-halogène, au reflux d'un mélange eau-diméthylsulfoxyde, selon la réaction de Krapcho décrite dans A.P. *Synthesis,* **1982**, 805, 893 pour conduire au composé de formule générale (Ic) selon le schéma réactionnel suivant : 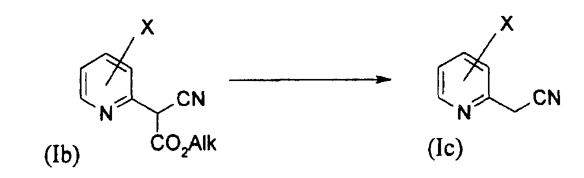 L'halogénure de sodium sera préférentiellement utilisé dans le cadre de la présente invention.
   * soit par l'acétonitrile pour mener directement au composé de formule générale (Ic) selon le schéma réactionnel suivant : 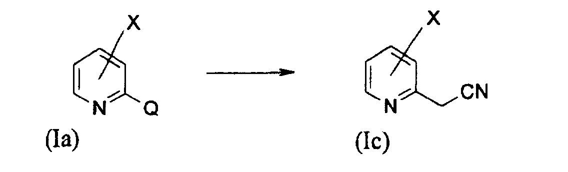
- une seconde étape consistant en la réduction du composé de formule générale (Ic) en pyridyl-éthanamine de formule générale (Id) (ou son sel d'ammonium correspondant selon que le milieu est acide ou non) sous pression d'hydrogène en présence d'un catalyseur métallique dans un solvant protique selon le schéma réactionnel suivant : 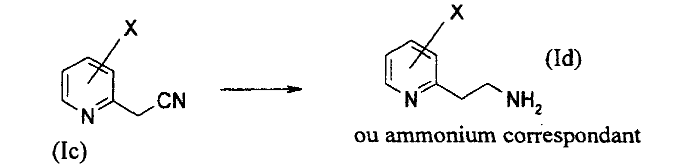 On préférera comme catalyseur métallique un catalyseur à base de nickel, de platine ou de palladium.
- une troisième étape consistant à convertir le composé de formule générale (Id) en composé de formule générale (I) par réaction avec un halogénure de benzoyle en présence d'une base selon le schéma réactionnel suivant : 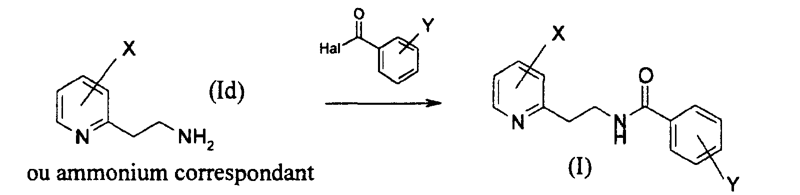
ou ammonium correspondant Le chlorure de benzoyle sera préférentiellement utilisé dans le cadre de la présente invention.

La présente invention a également pour objet une composition fongicide comprenant un composé de formule générale (I). Ainsi, la présente invention fournit une composition fongicide comprenant, comme matière active, un composé tel que défini précédemment, ainsi qu'un support acceptable en agrochimie.
Par le terme "support", on désigne, dans le présent exposé, une matière organique ou minérale, naturelle ou synthétique, avec laquelle la matière active est combinée pour faciliter son application sur les parties de la plante. Ce support est donc généralement inerte et il doit être acceptable en agriculture. Le support peut être solide (argiles, silicates naturels ou synthétiques, silice, résines, cires, engrais solides) ou liquide (eau, alcools, notamment le butanol, solvants organiques, huiles minérales et végétales et leurs dérivés). Des mélanges de tels supports peuvent être également utilisés.
D'une façon générale, la composition selon l'invention peut comprendre de 0,05 à 99% (en poids) de matière active.
La composition selon la présente invention peut également contenir, de manière optionnelle, un ou plusieurs agents tensio-actifs.
Par agent tensioactif, on entend, au sens de la présente invention, tout agent émulsionnant, dispersant ou mouillant de type ionique ou non ionique ou un mélange de tels agents tensioactifs. On peut citer par exemple des sels d'acides polyacryliques, des sels d'acides lignosulfoniques, des sels d'acides phénolsulfoniques ou naphtalènesulfoniques, des polycondensats d'oxyde d'éthylène sur des alcools gras ou sur des acides gras ou sur des amines grasses, des phénols substitués (notamment des alkylphénols ou des arylphénols), des sels d'esters d'acides sulfosucciniques, des dérivés de la taurine (notamment des alkyltaurates), des esters phosphoriques d'alcools ou de phénols polyoxyéthylés, des esters d'acides gras et de polyols, les dérivés à fonction sulfates, sulfonates et phosphates des composés précédents. La présence d'au moins un agent tensioactif est généralement indispensable lorsque la matière active et/ou le support inerte ne sont pas solubles dans l'eau et que l'agent vecteur de l'application est l'eau.
La composition selon l'invention peut prendre la forme d'assez nombreux types de formulations parmi lesquelles on peut citer les solutions huileuses, les concentrés émulsionnables, les poudres mouillables, les formulations fluides et notamment les suspensions aqueuses ou les émulsions aqueuses, les granulés, les poudres, les aérosols, les formulations fumigènes dont les formulations fumigènes auto combustibles ou les formulations fumigènes à réaction chimique, les formulations pour nébulisation notamment les formulations pour brumisation, les formulations à très bas volume, les pâtes, les émulsions, les suspensions concentrées, de même que d'éventuels mélanges, associations ou combinaisons de ces différentes formes.
De plus, la composition selon la présente invention peut comprendre simultanément, en plus du composé de formule générale (I), une ou plusieurs autres matières actives, par exemple des composés connus pour leurs propriétés de régulateur de croissance des plantes, pour leurs propriétés herbicides, fongicides, insecticides ou acaricides. La composition selon la présente invention peut également être utilisé de manière alternée ou séquentielle avec l'autre matière active.
La préparation des compositions selon la présente invention s'effectuera selon des moyens conventionnels et connus de l'homme du métier.

Les compositions selon la présente invention peuvent être utilisées pour combattre à titre préventif ou curatif contre les maladies phytopathogènes des plantes. Ainsi, la présente invention a également pour objet une méthode de traitement à titre préventif ou curatif contre les maladies phytopathogènes des plantes au moyen d'une quantité efficace et non phytotoxique d'une composition décrite précédemment.
Par 'quantité efficace et non phytotoxique', on entend, au sens de la présente invention, une quantité de composition selon l'invention suffisante pour permettre le contrôle ou la destruction des champignons présents ou susceptibles d'apparaître sur les cultures, et n'entraînant pour lesdites cultures aucun symptôme notable de phytotoxicité. Une telle quantité est susceptible de varier dans de larges limites selon le champignon à combattre, le type de culture, les conditions climatiques, et les composés compris dans la composition fongicide selon l'invention. Cette quantité peut être déterminée par des essais systématiques au champ, à la portée de l'homme du métier.
Au sens de la présente invention, on entend par méthode de traitement des plantes toute application d'une composition selon l'invention par des moyens connus de l'homme de métier sur le sol où poussent ou où sont susceptibles de pousser les plantes, ainsi que le traitement des plantes elles-mêmes.

La méthode de traitement des plantes selon l'invention peut être mise en oeuvre pour le traitement du matériel de propagation, par exemple les graines, les tubercules et les rhizomes, ainsi que les semences, les semis ou les semis de repiquage, les plants ou les plants de repiquage.
Cette méthode peut également être mise en oeuvre pour le traitement des racines.
Cette méthode peut aussi être mise en oeuvre pour le traitement des parties aériennes des plantes, par exemple les troncs, les tiges, les feuilles, les fleurs et les fruits.
Parmi les végétaux pouvant être traités selon l'invention, on peut citer : le coton; le lin; la vigne; les cultures fruitières telles que *Rosaceae* (notamment les fruits à pépin comme les pommes et les poires, les fruits à noyaux comme les abricots, les amandes et les pêches), *Ribesioidae, Juglandaceae, Betulaceae, Anacardiaceae, Fagaceae, Moraceae, Oleaceae, Actinidaceae, Lauraceae, Musaceae* (notamment les bananiers et les plantins), *Rubiaceae, Theaceae, Sterculiceae, Rutaceae* (notamment les citrons, les oranges et les pamplemousses); les cultures légumières telles que *Solanaceae* (notamment les tomates), *Liliaceae, Asteraceae* (notamment les laitues), *Umbelliferae, Cruciferae, Chenopodiaceae, Cucurbitaceae, Papilionaceae* (notamment les pois), *Rosaceae* (notamment les fraises); les grandes cultures telles que *Graminae* (notamment le maïs, les céréales comme le blé, le riz, l'orge et le triticale), *Asteraceae* (notamment le tournesol), *Cruciferae* (notamment le colza), *Papilionaceae* (notamment le soja), *Solanaceae* (notamment la pomme de terre), *Chenopodiaceae* (notamment la betterave); les cultures horticoles et forestières; ainsi que les homologues génétiquement modifiés de ces cultures.
On peut citer comme exemples non limitatifs de végétaux et de maladies pouvant toucher ces végétaux et susceptibles d'être traités par la méthode selon la présente invention :
- le blé, en ce qui concerne la lutte contre les maladies suivantes des semences : les fusarioses *(Microdochium nivale* et *Fusarium roseum),* les caries *(Tilletia caries, Tilletia controversa* ou *Tilletia indica),* la septoriose *(Septoria nodorum) ;* le charbon nu *(Ustilago tritici);*
- le blé, en ce qui concerne la lutte contre les maladies suivantes des parties aériennes de la plante : le piétin-verse *(Tapesia yallundae, Tapesia acuiformis),* le piétin-échaudage *(Gaeumannomyces graminis),* la fusariose du pied (F. *culmorum, F. graminearum),* la fusariose des épis (F. *culmorum, F. graminearum, Microdochium nivale),* le rhizoctone *(Rhizoctonia cerealis),* l'oïdium *(Erysiphe graminis forma specie tritici),* les rouilles *(Puccinia striiformis* et *Puccinia recondita)* et les septorioses *(Septoria tritici* et *Septoria nodorum),* l'helminthosporiose *(Drechslera tritici-repentis) ;*
- le blé et l'orge, en ce qui concerne la lutte contre les maladies bactériennes et virales, par exemple la jaunisse nanisante de l'orge ;
- l'orge, en ce qui concerne la lutte contre les maladies suivantes des semences : les helminthosporioses *(Pyrenophora graminea, Pyrenophora teres* et *Cochliobolus sativus),* le charbon nu *(Ustilago nuda)* et les fusarioses *(Microdochium nivale* et *Fusarium roseum);*
- l'orge, en ce qui concerne la lutte contre les maladies suivantes des parties aériennes de la plante : le piétin-verse *(Tapesia yallundae),* les helminthosporioses *(Pyrenophora teres* et *Cochliobolus sativus),* l'oïdium *(Erysiphe graminis forma specie hordei),* la rouille naine *(Puccinia hordei)* et la rhynchosporiose *(Rhynchosporium secalis) ;*
- la pomme de terre, en ce qui concerne la lutte contre les maladies du tubercule (notamment *Helminthosporium solani, Phoma tuberosa, Rhizoctonia solani, Fusarium solani),* le mildiou *(Phytophthora infestans)* et certaines viroses (virus Y) ;
- la pomme de terre en ce qui concerne la lutte contre les maladies du feuillage suivantes : l'altemariose *(Alternaria solani),* le mildiou *(Phytophthora infestans) ;*
- le coton, en ce qui concerne la lutte contre les maladies suivantes des jeunes plantes issues des semences : les fontes de semis et les nécroses du collet *(Rhizoctonia solani, Fusarium oxysporum),* la pourriture noire des racines *(Thielaviopsis basicola) ;*
- les cultures protéagineuses, par exemple le pois, en ce qui concerne la lutte contre les maladies suivantes des semences : l'anthracnose *(Ascochyta pisi, Mycosphaerella pinodes),* la fusariose *(Fusarium oxysporum),* la pourriture grise *(Botrytis cinerea),* le mildiou *(Peronospora pisi) ;*
- les cultures oléagineuses, par exemple le colza, en ce qui concerne la lutte contre les maladies suivantes des semences : *Phoma lingam, Alternaria brassicae* et *Sclerotinia sclerotiorum ;*
- le maïs, en ce qui concerne la lutte contre les maladies des semences : *(Rhizopus* sp., *Pénicillium* sp., *Trichoderma* sp., *Aspergillus* sp. et *Gibberella fujikuroi);*
- le lin, en ce qui concerne la lutte contre la maladie des semences : *Alternaria linicola ;*
- les arbres forestiers, en ce qui concerne la lutte contre les fontes de semis *(Fusarium oxysporum, Rhizoctonia solani);*
- le riz en ce qui concerne la lutte contre les maladies suivantes des parties aériennes : la pyriculariose *(Magnaporthe grisea),* le rhizoctone *(Rhizoctonia solani);*
- les cultures légumières en ce qui concerne la lutte contre les maladies suivantes des semis ou des jeunes plants issus de semences : les fontes de semis et les nécroses du collet *(Fusarium oxysporum, Fusarium roseum, Rhizoctonia solani, Pythium sp.);*
- les cultures légumières en ce qui concerne la lutte contre les maladies suivantes des parties aériennes : la pourriture grise (Botrytis sp.), les oïdiums (notamment *Erysiphe cichoracearum, Sphaerotheca fuliginea, Leveillula taurica),* les fusarioses *(Fusarium oxysporum, Fusarium roseum),* les cladosporioses *(Cladosporium sp.),* les altemarioses *(Alternaria sp.),* les anthracnoses *(Colletotrichum sp.),* les septorioses *(Septoria sp.),* le rhizoctone *(Rhizoctonia solani),* les mildious (par exemple *Bremia lactucae, Peronospora sp., Pseudoperonospora sp, Phytophthora sp);*
- les arbres fruitiers en ce qui concerne les maladies des parties aériennes : la moniliose *(Monilia fructigenae, M. laxa),* la tavelure *(Venturia inaequalis),* l'oïdium *(Podosphaera leucotricha);*
- la vigne en ce qui concerne les maladies du feuillage : notamment la pourriture grise *(Botrytis cinerea),* l'oïdium *(Uncinula necator),* le black-rot *(Guignardia biwelli),* le mildiou *(Plasmopara viticola);*
- la betterave en ce qui concerne les maladies suivantes des parties aériennes : la cercosporiose *(Cercospora beticola),* l'oïdium *(Erysiphe beticola),* la ramulariose *(Ramularia beticola).*

Les exemples de composés qui suivent sont mentionnés dans le but d'illustrer l'invention mais ne doivent en aucun cas être considérés comme limitatifs de celle-ci. Dans les exemples suivants, M+1 représente le pic ionique moléculaire plus 1 u.m.a (unité de masse atomique) observé à la spectrométrie de masse.

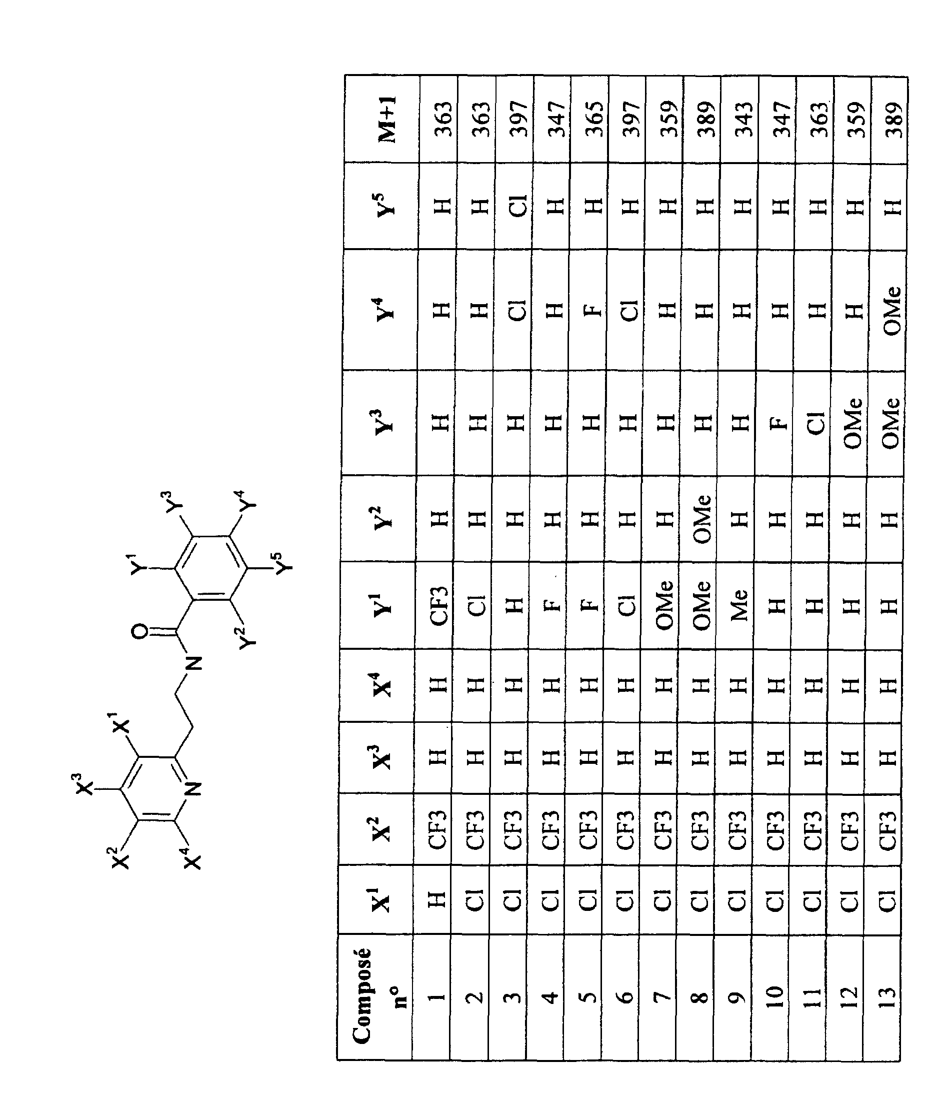

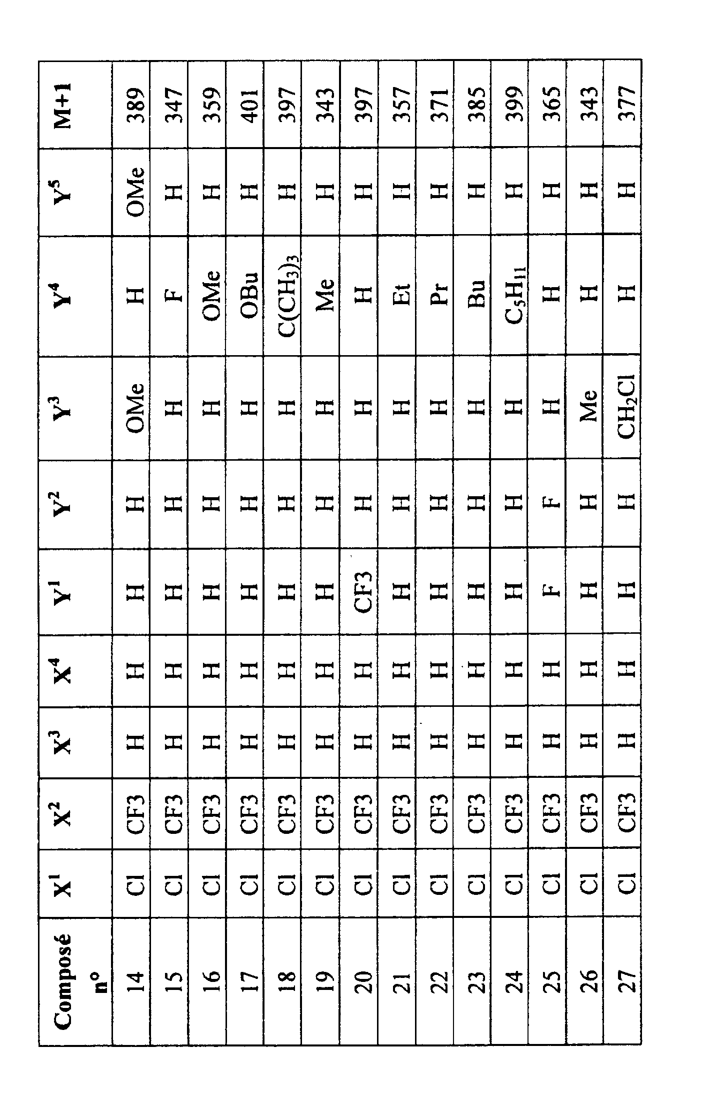

Les exemples de préparation de composés qui suivent sont mentionnés dans le but d'illustrer l'invention mais ne doivent en aucun cas être considérés comme limitatifs de celle-ci.

### Préparation du methyl [3-chloro-5-(trifluoromethyl)-2-pyridinyl](cyano)acetate:

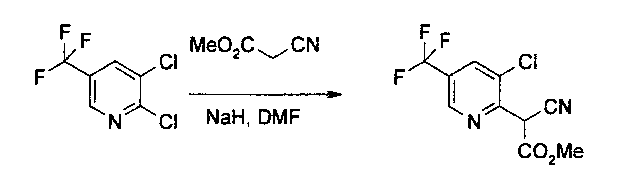

### Procédure :

Sous argon, 116 g d'hydrure de sodium, 60% en dispersion dans l'huile (2.91 mol, 1.8 éq.) sont suspendus dans 3L de DMF. La suspension est refroidie dans un bain d'eau glacée. 160 g ( 1.616 mol, 1.0 éq.) de méthyl cyanoacetate en solution dans 200 mL de DMF sont additionnés sous agitation au goutte à goutte. A la fin du dégagement gazeux, 350g ( 1.616 mol, 1.0 eq) of 2,3-dichloro-5-(trifluoromethyl)-pyridine sont additionnés sous agitation. Le mélange est agité toute une nuit à température ambiante. 50mL de méthanol sont ajoutés. Le milieu réactionnel est versé dans 5L d'eau. Le pH est ajusté à 3-4 avec de l'acide chlorhydrique concentré. Le précipité jaune de methyl [3-chloro-5-(trifluoromethyl)-2-pyridinyl](cyano)acetate qui se forme est filtré et lavé à l'eau et au pentane.

### Préparation du [3-chloro-5-(trifluoromethyl)-2-pyridinyl] acetonitrile:

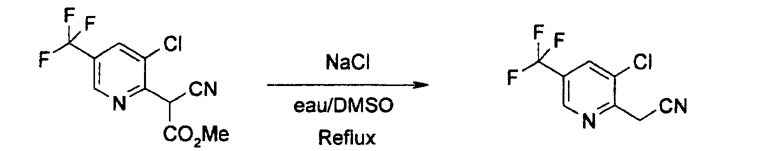

### Procédure :

314g (1.13 mol, 1eq.) de methyl [3-chloro-5-(trifluoromethyl)-2-pyridinyl](cyano)acetate et 22g ( 0.38mol, 0.33 eq.) de chlorure de sodium sont dissous dans une solution de 44 mL d'eau et 1.1L de dimethyl sulfoxyde. Le milieu réactionnel est agité et chauffé à 160°C. A la fin du dégagement gazeux, le milieu est refroidi jusqu'à température ambiante. 1L d'eau et 0.5L de dichlorométhane sont additionnés. Après séparation, la phase aqueuse est extraite deux fois avec 0.5L de dichlorométhane. La phase organique est lavée deux fois avec 0.5L d'eau et séchée sur sulfate de magnésium. Après concentration, le produit brut est dilué dans 100mL de dichlorométhane et élué avec un mélange acétate d'éthyle/heptane (20/80) sur un lit de silice. Le filtrat est concentré pour produire le [3-chloro-5-(trifluoromethyl)-2-pyridinyl] acetonitrile.

### Préparation du 2-[3-chloro-5-(trifluoromethyl)-2-pyridinyl] ethanamine acetate:

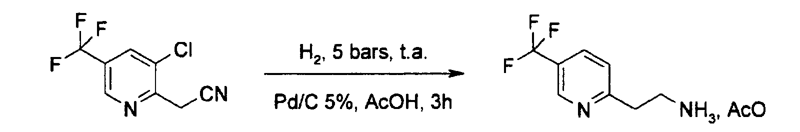

### Procédure :

113g de [3-chloro-5-(trifluoromethyl)-2-pyridinyl] acetonitrile (0.51mol, leq.) sont dilués dans 2.5L d'acide acétique. 30g de palladium (5% sur charbon) sont additionnés. Le milieu réactionnel est agité à température ambiante sous une pression d'hydrogène de 5 bars. L'avancement de la réaction est suivi par CCM, lorsque le [3-chloro-5-(trifluoromethyl)-2-pyridinyl] acetonitrile est entièrement consommé, le milieu est filtré sur un lit de célite, puis concentré à sec pour produire le 2-[3-chloro-5-(trifluoromethyl)-2-pyridinyl] ethanamine acetate.

### Préparation du N-{2- [3-chloro-5-(trifluoromethyl)-2-pyridinyl]ethyl}benzamides:

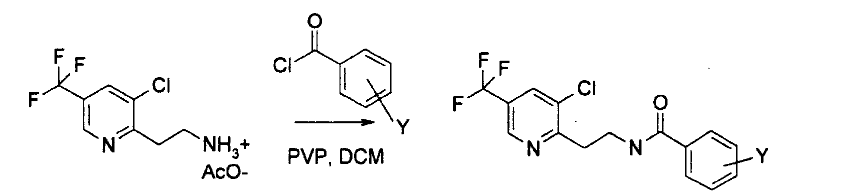

### Procédure :

0.100g de 2-[3-chloro-5-(trifluoromethyl)-2-pyridinyl] ethanamine acetate (0.00037mol, 1.0 éq.) sont dilués dans 3mL de dichlorométhane. 0.500g de poly-4-vinyl-pyridine sont ajoutés. Le mélange est agité à température ambiante pendant une demi-heure.
1.2 équivalents du chlorure d'acyle souhaité sont additionnés. Le milieu réactionnel est agité à température ambiante pendant une nuit, filtré, et concentré à sec. Le produit brut est ensuite purifié par HPLC phase inverse. L'amide correspondant est obtenu.

Les activités biologiques suivantes ont été testées afin d'établir une comparaison entre l'activité des composés de formule générale (I) selon la présente invention et un composé décrit dans la demande de brevet WO 01/11965 sur un certain nombre de maladies fongiques.

### Test in vivo de l'activité sur Alternaria brassicae (altemariose des crucifères)

Une solution aqueuse de la matière active à tester est préparée à la concentration de 2g/l par broyage dans la solution suivante :
- eau
- Tween 80 dilué à 10% dans l'eau : 5ml/mg de matière active
- Argile à la quantité suffisante pour que matière active + argile égale 100mg.

La solution aqueuse est diluée avec de l'eau pour obtenir la concentration souhaitée.
Des radis de la variété Pernot sont semés dans un substrat 50/50 pozzolane et tourbe et maintenus à 18-22°C. Le traitement est effectué par pulvérisation de la suspension aqueuse. Les plantes témoin non traitées sont pulvérisées avec de l'eau. 24 heures après le traitement, les plantes sont inoculées par pulvérisation d'une solution de spores (40000 spores/ml) de *Alternaria brassicae* provenant d'une culture de 12 jours.
Les plants de radis sont alors maintenus à 18-20°C en atmosphère humide. L'efficacité des produits est évaluée par rapport aux plantes témoin après sept à huit jours d'incubation.

### Test in vivo de l'activité sur Botrytis cinerea sur concombre

Une solution aqueuse de la matière active à tester est préparée à la concentration de 2g/l par broyage dans la solution suivante :
- eau
- Tween 80 dilué à 10% dans l'eau : 5ml/mg de matière active
- Argile à la quantité suffisante pour que matière active + argile égale 100mg.

La solution aqueuse est diluée avec de l'eau pour obtenir la concentration souhaitée.

Des concombre de la variété Marketer sont semés dans un substrat 50/50 pozzolane et tourbe et maintenus à 18-22°C. Le traitement est effectué par pulvérisation de la suspension aqueuse. Les plantes témoin non traitées sont pulvérisées avec de l'eau.
24 heures après le traitement, les plantes sont inoculées par pulvérisation d'une solution de spores (150000spores/ml) de *Botrytis cinerea* provenant d'une culture de 15 jours.
Les plants de concombre sont alors maintenus à 11-15°C en atmosphère humide. L'efficacité des produits est évaluée par rapport aux plantes témoin après sept à huit jours d'incubation.

### Test in vivo de l'activité sur Pyrenophora teres (Helminthosporiose de l'orge)

Une solution aqueuse de la matière active à tester est préparée à la concentration de 2g/l par broyage dans la solution suivante :
- eau
- Tween 80 dilué à 10% dans l'eau : 5ml/mg de matière active
- Argile à la quantité suffisante pour que matière active + argile égale 100mg.

La solution aqueuse est diluée avec de l'eau pour obtenir la concentration souhaitée.
Des orges de la variété Express sont semés dans un substrat 50/50 pozzolane et tourbe et maintenus à 12°C. Le traitement est effectué au stade 1 feuille (10 cm) par pulvérisation de la suspension aqueuse. Les plantes témoin non traitées sont pulvérisées avec de l'eau.
24 heures après le traitement, les plantes sont inoculées par pulvérisation d'une solution de spores (10000 spores/ml) de *Pyrenophora teres* provenant d'une culture de 10 jours.
Les plants de radis sont alors maintenus à 18°C en atmosphère humide. L'efficacité des produits est évaluée par rapport aux plantes témoin après huit à quinze jours d'incubation.

### Test in vivo de l'activité sur Septoria tritici (septoriose du blé)

Une solution aqueuse de la matière active à tester est préparée à la concentration de 2g/l par broyage dans la solution suivante :
- eau
- Tween 80 dilué à 10% dans l'eau : 5ml/mg de matière active
- Argile à la quantité suffisante pour que matière active + argile égale 100mg.

La solution aqueuse est diluée avec de l'eau pour obtenir la concentration souhaitée.
Des blé de la variété Scipion sont semés dans un substrat 50/50 pozzolane et tourbe et maintenus à 12°C. Le traitement est effectué au stade 1 feuille (10cm) par pulvérisation de la suspension aqueuse. Les plantes témoin non traitées sont pulvérisées avec de l'eau.
24 heures après le traitement, les plantes sont inoculées par pulvérisation d'une solution de spores (500000 spores/ml) de *Septoria tritici* d'une culture de 7 jours.
Les plantules de blé sont alors maintenues à 18-20°C en atmosphère humide pendant 72 heures puis à 90% d'humidité relative. L'efficacité des produits est évaluée par rapport aux plantes témoin 21 à 28 jours après la contamination.

L'efficacité des molécules est estimée à 500 g/ha par le pourcentage de contrôle par rapport à des plantes non traitées. Sous ces conditions, une bonne efficacité est définie par plus de 80% d'efficacité. Une efficacité moyenne est définie par une efficacité comprise entre 50 et 80%. Une faible efficacité est définie par une efficacité comprise entre 10 et 50% et une efficacité nulle par moins de 10%.
A la concentration de 500 g/ha, les composés suivants ont montré une efficacité bonne à moyenne contre les pathogènes fongiques :
*Alternaria brassicae :* 2, 4, 6, 7, 9,13, 14, 20, 25, 27
*Botrytis cinerea :* 2, 7, 9, 20, 25
*Pyrenophora teres :* 2, 4, 5, 6, 7, 9, 20, 25, 27
*Septoria tritici :* 2, 4, 5, 6, 7, 16, 18, 20, 21, 22, 23, 24, 25

Dans ces conditions le N-{[3-chloro-5-(trifluorométhyl)-2-pyridinyl] éthyl}benzamide couvert par la demande de brevet WO 01/11965 a montré une efficacité faible sur *Septoria tritici* et *Pyrenophora teres* et nulle sur *Botrytis cinerea* et *Allemand brassicae* à 500 g/ha.

## Revendications

1. Composé de formule générale (I): 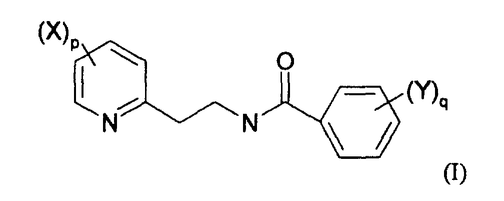 dans laquelle :
- p est un entier égal à 1, 2, 3 ou 4;
- q est une entier égal à 1, 2, 3, 4 ou 5;
- chaque substituant X est choisi indépendamment des autres dans le groupe consistant en halogène, alkyle et halogénoalkyle;
- chaque substituant Y est choisi indépendamment des autres dans le groupe consistant en halogène, alkyle, halogénoalkyle, alkoxy, phenoxy, alkylthio, dialkylamino, acyle, cyano, nitro, alkylsulphonyle, phénylsulphonyle, benzylsulphonyle et S-phényle substitué par un halogène;
à l'exception du N-{[3-chloro-5-(trifluorométhyl)-2-pyridinyl] éthyl}-2,6-dichlorobenzamide.

2. Composé selon la revendication 1, **caractérisé en ce que** p est égal à 2.

3. Composé selon la revendication 2, **caractérisé en ce que** les substituants X sont positionnés comme suit :

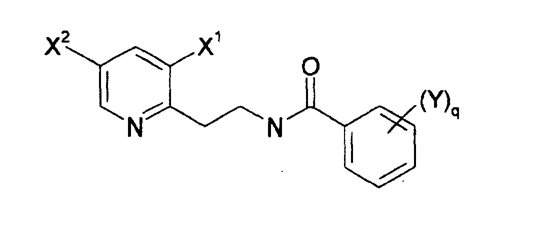

4. Composé selon l'une quelconque des revendications 1 à 4, **caractérisé en ce que** q est choisi égal à 1 ou 2, le(s) substituant(s) Y étant positionné(s) en position ortho du cycle benzénique.

5. Composé selon la revendication 4 , **caractérisé en ce qu'**il répond à la formule générale (I'):

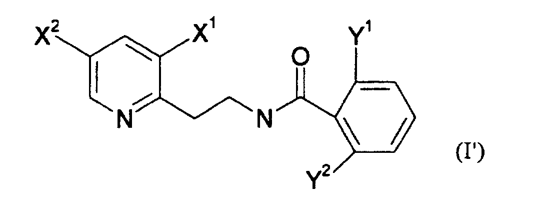

6. Composé selon la revendication 5, **caractérisé en ce que** X¹ est un halogène et X² est un halogénoalkyle.

7. Composé selon la revendication 4, **caractérisé en ce qu'**il répond à la formule générale (I")

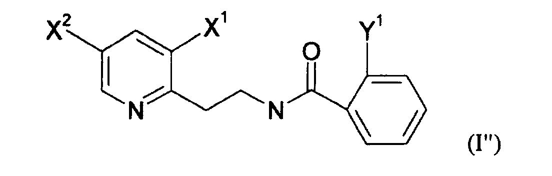

8. Composé selon la revendication 7, **caractérisé en ce qu'**il possède les caractéristiques suivantes, prises isolément ou en combinaison :
- X¹ est choisi comme étant un halogène et X² est choisi comme étant un halogénoalkyle ;
- Y¹ est choisi comme étant un halogénoalkyle.

9. Composé selon la revendication 8, **caractérisé en ce que** :
- X¹ est CI;
- X² est CF₃;
- Y¹ est CF₃.

10. Procédé de préparation du composé selon l'une quelconque des revendications 1 à 9 **caractérisé en ce qu'**il comprend les étapes suivantes :
- une première étape consistant à faire réagir en présence d'une base en solvant polaire aprotique, un composé de formule générale (Ia) pour le substituer sélectivement en position 2 :
* soit par un groupement de type cyanoacétate d'alkyle (NC-CH₂-CO₂Alk) pour conduire à un composé de formule générale (Ib) selon le schéma réactionnel suivant : 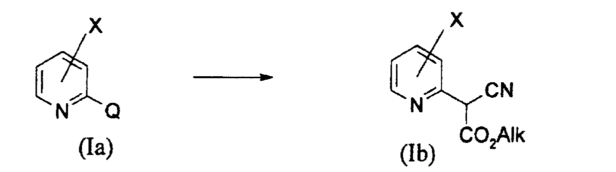
où :
- X est tel que défini où Y est tel que défini dans l'une quelconque des revendications 1 à 9;
- Alk représente un radical alkyle;
- Q est un radical nucléofuge;
le composé de formule générale (Ib) ainsi obtenu étant alors décarboalkoxylé en présence d'un halogénure alcalin tel que Li-Halogène, K-Halogène ou Na-halogène, au reflux d'un mélange eau-diméthylsulfoxyde, selon la réaction de Krapcho décrite dans A.P. *Synthesis,* **1982**, 805, 893 pour conduire au composé de formule générale (Ic) selon le schéma réactionnel suivant : 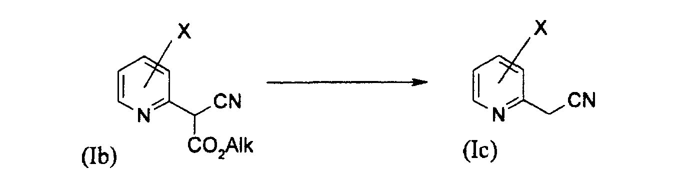
* soit par l'acétonitrile pour mener directement au composé de formule générale (Ic) selon le schéma réactionnel suivant : 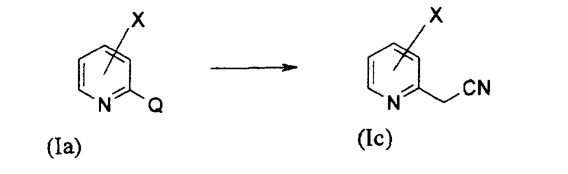
- une seconde étape consistant en la réduction du composé de formule générale (Ic) en pyridyl-éthanamine de formule générale (Id) (ou son sel d'ammonium correspondant selon que le milieu est acide ou non) sous pression d'hydrogène en présence d'un catalyseur métallique dans un solvant protique selon le schéma réactionnel suivant : 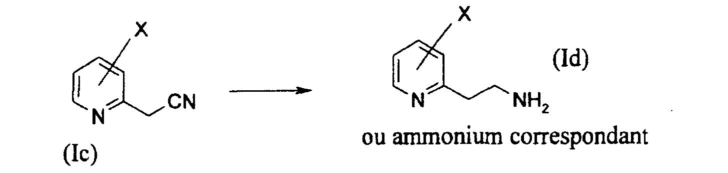
- une troisième étape consistant à convertir le composé de formule générale (Id) en composé de formule générale (I) par réaction avec un halogénure de benzoyle en présence d'une base selon le schéma réactionnel suivant : 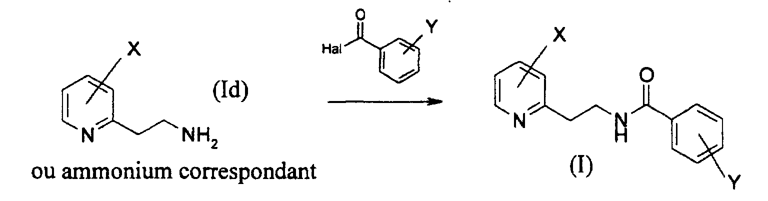 où Y est tel que défini dans l'une quelconque des revendications 1 à 9.

11. Procédé selon la revendication 10, **caractérisé en ce que** le radical nucléofuge Q est un halogène ou le trifluorométhanesulfonate,

12. Composition fongicide comprenant comme matière active un composé tel que défini dans l'une quelconque des revendications 1 à 9, ainsi qu'un support acceptable en agrochimie.

13. Composition selon la revendication 12 comprenant en plus un agent tensioactif.

14. Composition selon la revendication 12 ou 13, **caractérisé en ce qu'**elle comprend de 0,05 à 99% (en poids) de matière active.

15. Méthode de traitement à titre préventif ou curatif contre les maladies phytopathogènes des plantes au moyen d'une quantité efficace et non phytotoxique d'une composition selon l'une quelconque des revendications 12 à 14.

## Revendications modifiées

### Revendications modifiées conformément à la règle 86(2) CBE.

**1.** Composé de formule générale (I): 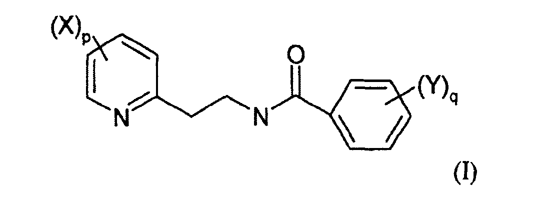 dans laquelle :
- p est un entier égal à 1, 2, 3 ou 4;
- q est une entier égal à 1, 2, 3, 4 ou 5;
- chaque substituant X est choisi indépendamment des autres dans le groupe consistant en halogène, alkyle et halogénoalkyle, l'un au moins des substituants étant un halogénoalkyle;
- chaque substituant Y est choisi indépendamment des autres dans le groupe consistant en halogène, alkyle, halogénoalkyle, alkoxy, phenoxy, alkylthio, dialkylamino, acyle, cyano, nitro, alkylsulphonyle, phénylsulphonyle, benzylsulphonyle et S-phényle substitué par un halogène;
à l'exception du N-{2-[3-chloro-5-(trifluorométhyl)-2-pyridinyl] éthyl}-2,6-dichlorobenzamide.

**2.** Composé selon la revendication 1, **caractérisé en ce que** p est égal à 2.

**3.** Composé selon la revendication 2, **caractérisé en ce que** les substituants X sont positionnés comme suit :
